(19) Europäisches Patentamt
European Patent Office
Office européen des brevets

(11) Veröffentlichungsnummer: **0 074 511**
**B1**

(12) # EUROPÄISCHE PATENTSCHRIFT

(45) Veröffentlichungstag der Patentschrift: **09.12.87**

(51) Int. Cl.⁴: **C 07 D 285/00,** A 01 N 43/72

(21) Anmeldenummer: **82107623.9**

(22) Anmeldetag: **20.08.82**

(54) **2H-1,2,4,6-Thiatriazin(3)on-1,1-dioxide, Verfahren zu ihrer Herstellung und ihre Verwendung zur Bekämpfung unerwünschten Pflanzenwuchses.**

(30) Priorität: **28.08.81 DE 3134141**

(43) Veröffentlichungstag der Anmeldung: **23.03.83 Patentblatt 83/12**

(45) Bekanntmachung des Hinweises auf die Patenterteilung: **09.12.87 Patentblatt 87/50**

(84) Benannte Vertragsstaaten: **AT BE CH DE FR GB IT LI NL**

(56) Entgegenhaltungen:
**EP - A - 0 025 113**
**DE - A - 2 508 832**

**R. WEGLER Chemie der Pflanzenschutz- und Schädlingsbekämpfungsmittel, Band 5, Springer-Verlag Berlin, 1977, S. 356, 357**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Acker, Rolf-Dieter, Dr., Tuchbleiche 8, D-6906 Leimen (DE)**
Erfinder: **Wuerzer, Bruno, Dr. Dipl.-Landwirt, Ruedigerstrasse 13, D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft 2H-1,2,4,6-Thiatriazin-(3)on-1,1-dioxide, ein Verfahren zu ihrer Herstellung, Herbizide, die diese Verbindungen als Wirkstoffe enthalten, sowie ein Verfahren zur Bekämpfung unerwünschter Pflanzenwuchses mit diesen Verbindungen.

Substituierte 5,6-Dihydro-1,2,4,6-thiatriazin(5)on-1,1-dioxide sind als Wirkstoffe für Pflanzenschutzmittel bekannt (DE-OS 1 946 262). Herbizid wirksame 5,6-Dihydro-1,2,4,6-thiatriazin(5)on-1,1-dioxide sind aus DE-OS 2 508 832 und DE-OS 2 933 889 bekannt. Ausserdem ist in J. Chem. Res (1977), 2813–2825 die Herstellung von

2-Methyl- und 2-Isopropyl-5-Methyl-2H-1,2,4,6-thiatriazin(3)-on-1,1-dioxid

ohne Angaben über die Verwendungsmöglichkeit dieser Verbindungen beschrieben. Es wird dort darauf hingewiesen, dass die Cyclisierung nur unter grossen Schwierigkeiten und mit beträchtlichem experimentellem Aufwand durchgeführt werden kann, wobei die Ausbeute nur 22 bzw. 26% beträgt.

2-Isopropyl-5-phenyl-2H-1,2,4,6-thiatriazin-(3)on-1,1-dioxid

wird durch Umsetzung von N-Carboethoxy-benzamidin und Isopropylaminosulfonylchlorid in Tetrahydrofuran bei −70 °C und Cyclisierung mit Natriumethylat in nur 18%iger Ausbeute erhalten (J. Chem. Res. (1977), 2826–2836).

Es wurde gefunden, dass 2H-1,2,4,6-Thiatriazin-(3)on-1,1-dioxidderivate der Formel

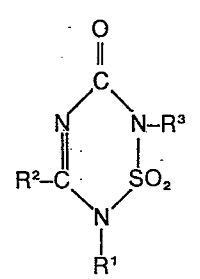

(I),

in der

$R^1$ Wasserstoff, ein Metallatom oder einen gegebenenfalls durch Alkyl oder Hydroxyalkyl mit jeweils 1 bis 10 Kohlenstoffatomen oder Cycloalkyl mit 3 bis 7 Kohlenstoffatomen substituiertem Ammoniumrest,

$R^2$ einen unverzweigten $C_1$–$C_{10}$- oder verzweigten $C_3$–$C_{10}$-ALkylrest, einen Cycloalkylrest mit 3 bis 7 Kohlenstoffatomen, einen halogen-substituierten Alkylrest mit 2 bis 4 Kohlenstoffatomen,
Methoxyethyl, Ethoxyethyl, 3-Methoxy-n-propyl,
Methoxyisopropyl, 3-Methoxy-n-butyl,
1-Methoxy-butyl-2, Ethoxy-tert-butyl,
Methoxy-tert-butyl, 2-Methoxy-butyl,
4-Methoxy-n-butyl, Methylmercapto-ethyl,
Ethylmercapto-ethyl, 3-Methylmercapto-n-propyl
3-Methylmercapto-n-butyl,
1-Methylmercapto-butyl-2,
Methylmercapto-tert-butyl,
2-Methylmercapto-n-butyl
oder einen gegebenenfalls durch Halogen oder Alkyl mit 1 bis 4 Kohlenstoffatomen substituierten Phenylrest und

$R^3$ Wasserstoff, einen unverzweigten $C_1$–$C_4$- oder verzweigten $C_3$–$C_4$-Alkylrest, einen Cycloalkylrest mit 3 bis 7 Kohlenstoffatomen, einen halogen-substituierten Alkylrest mit 2 bis 4 Kohlenstoffatomen,
Methoxyethyl, Ethoxyethyl, 3-Methoxy-n-propyl,
Methoxyisopropyl, 3-Methoxy-n-butyl,
1-Methoxy-butyl-2, Ethoxy-tert-butyl,
Methoxy-tert-butyl, 2-Methoxy-butyl oder
4-Methoxy-n-butyl
bedeuten,
mit der Massgabe, dass $R^2$ nicht Methyl bedeutet, wenn $R^1$ für Wasserstoff und $R^3$ für Methyl oder Isopropyl stehen, und mit der Massgabe, dass $R^2$ nicht Phenyl bedeutet, wenn $R^1$ für Wasserstoff und $R^3$ für Isopropyl stehen, neu sind und eine selektive herbizide Wirkung haben.

$R^1$ in Formel I bedeutet beispielsweise ein Alkalimetallatom, wie Natrium, Kalium, oder einen gegebenenfalls durch Alkyl oder Hydroxyalkyl mit jeweils 1 bis 10 Kohlenstoffatomen oder Cycloalkyl und 3 bis 7 Kohlenstoffatomen substituierten Ammoniumrest, wie Ammonium, Dimethylammonium, Triethanolammonium, Tridecylammonium, Trimethylammonium, Diisopropylmethylammonium, Diisopropylethylammonium, N-Methyl-N,N-diethanolammonium, Diisopropylammonium, Cyclohexylammonium, polyoxyethyliertes Dimethylammonium, Triethylammonium, N,N-Dimethyl-N-cyclohexylammonium.

$R^2$ in Formel I bedeutet unverzweigte Alkylreste mit 1 bis 10 Kohlenstoffatomen, vorzugsweise 2 bis 4 Kohlenstoffatomen, verzweigte Alkylreste mit 3 bis 10 Kohlenstoffatomen, vorzugsweise verzweigte Propyl- und Butylreste, wie
Ethyl, n-Propyl, Isopropyl, n-Butyl, Isobutyl,
sec-Butyl, tert.-Butyl, n-Pentyl, n-Hexyl,
Pentyl-3, 1,2-Dimethyl-n-propyl,
1,3-Dimethyl-n-butyl, 1-Ethyl-2-methyl-n-propyl,
1,2,2-Trimethyl-n-propyl, 1,2-Dimethyl-n-Hexyl,
tert.-Amyl,
einen Halogenalkylrest mit 2 bis 4 Kohlenstoffatomen, wie
2-Chlorethyl, 2-Chlor-n-propyl,
3-Chlor-n-propyl, 2-Chlorisopropyl,
1-Chlormethyl-n-propyl, 2-Chlorbutyl-3,
2-Chlor-2-methyl-n-propyl, 2-Fluorbutyl-3,
2-Fluor-2-methyl-n-propyl, 2-Fluorisopropyl,
Chlor-tert-butyl, 2,2,2-Trifluorethyl,
Methoxyethyl, Ethoxyethyl, 3-Methoxy-n-propyl,
Methoxyisopropyl, 3-Methoxy-n-butyl,
1-Methoxy-butyl-2, Ethoxy-tert-butyl,
Methoxy-tert-butyl, 2-Methoxy-butyl,
4-Methoxy-n-butyl, Methylmercapto-ethyl,
Ethylmercapto-ethyl, 3-Methylmercapto-n-propyl,
3-Methylmercapto-n-butyl,
1-Methylmercapto-butyl-2,
Methylmercapto-tert-butyl,
2-Methylmercapto-n-butyl,
einen Cycloalkylrest mit 3 bis 7 Kohlenstoffatomen, wie
Cyclopropyl, Cyclophentyl, Cyclohexyl,
Cycloheptyl,

oder einen gegebenenfalls durch Halogen, wie Fluor, Chlor, Brom, Jod, oder Alkyl mit 1 bis 4 Kohlenstoffatomen substituierten Phenylrest, wie Phenyl, 4-Chlorphenyl, 2,4-Dichlorphenyl, 4-Isopropylphenyl, 4-tert.-Butylphenyl, mit der Massgabe, dass $R^2$ nicht Methyl bedeutet, wenn $R^1$ für Wasserstoff und $R^3$ für Methyl oder Isopropyl stehen, und mit der Massgabe, dass $R^2$ nicht Phenyl bedeutet, wenn $R^1$ für Wasserstoff und $R^3$ für Isopropyl stehen.

$R^3$ in Formel I bedeutet Wasserstoff, einen unverzweigten Alkylrest mit 1 bis 4 Kohlenstoffatomen, einen verzweigten Alkylrest mit 3 oder 4 Kohlenstoffatomen, wie Methyl, Ethyl, n-Porpyl, Isopropyl, n-Butyl, Isobutyl, sec-Butyl, tert.-Butyl, einen durch Halogen, wie Fluor, Chlor, Brom, Jod, substituierten Alkylrest mit 2 bis 4 Kohlenstoffatomen, wie
2-Chlorethyl, 2-Chlor-n-propyl,
3-Chlor-n-propyl, 2-Chlorisopropyl,
1-Chlormethyl-n-propyl, 2-Chlorbutyl-3,
2-Chlor-2-methyl-n-propyl, 2-Fluorbutyl-3,
2-Fluor-2-methyl-n-propyl, 2-Fluorisopropyl,
Chlor-tert-butyl, 2,2,2-Trifluorethyl,
Methoxyethyl, Ethoxyethyl, 3-Methoxy-n-propyl,
Methoxyisopropyl, 3-Methoxy-n-butyl,
1-Methoxy-butyl-2, Ethoxy-tert-butyl,
Methoxy-tert-butyl, 2-Methoxy-butyl,
4-Methoxy-n-butyl,
oder einen Cycloalkylrest mit 3 bis 7 Kohlenstoffatomen, wie Cyclopropyl, Cyclopentyl, Cyclohexyl, Cycloheptyl.

Bevorzugte Verbindungen der Formel I sind solche, bei denen $R^1$ für Wasserstoff, $R^2$ für Alkyl mit 1 bis 4 Kohlenstoffatomen und $R^3$ für Alkyl mit 2 bis 4 Kohlenstoffatomen, ausgenommen Isopropyl, steht.

Man erhält die 2H-1,2,4,6-Thiatriazin(3)-on-1,1-dioxide der Formel I durch Umsetzung von N-Carboalkoxy-amidinen der Formel

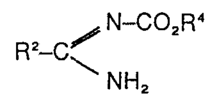
(III),

in der $R^2$ die oben genannten Bedeutungen hat und $R^4$ einen Alkylrest mit 1 bis 4 Kohlenstoffatomen bedeutet, mit einem Aminosulfonylhalogenid der Formel

$$R^3\text{–}NHSO_2Y \quad (IV),$$

in der $R^3$ die oben genannten Bedeutungen hat und Y für Fluor oder Chlor steht, gegebenenfalls in Gegenwart eines Säureakzeptors und eines inerten organischen Lösungsmittels bei einer Temperatur von −20 bis +80 °C zu Sulfondiamiden der Formel

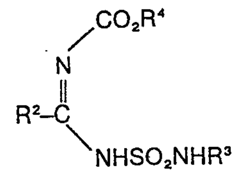
(II),

in der $R^2$, $R^3$ und $R^4$ die oben genannten Bedeutungen haben und Cyclisierung dieser Sulfondiamide in Gegenwart einer basischen Verbindung bei einer Temperatur von 0 bis 100 °C.

N-Carboalkoxy-amidine der Formel III sind teilweise bekannt oder lassen sich nach bekannten Methoden herstellen.

Verwendet man N-Carbomethoxy-acetamidin und Isopropylaminosulfonylchlorid als Ausgangsmaterialien, so kann der Reaktionsablauf durch folgendes Formelschema wiedergegeben werden:

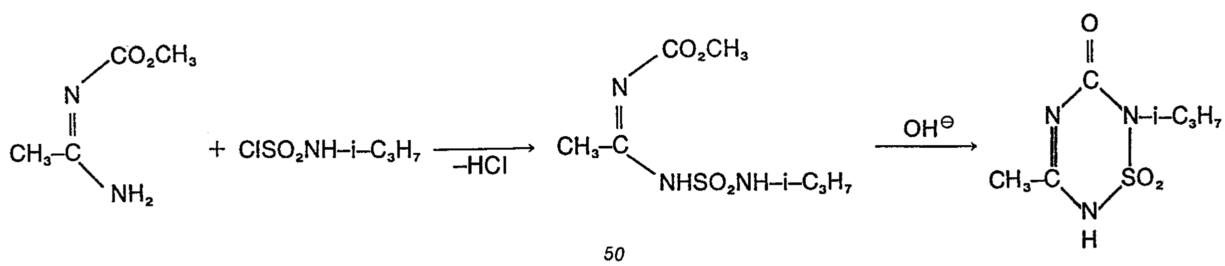


Die Umsetzung des N-Carboalkoxy-amidins der Formel III mit dem Aminosulfonylhalogenid der Formel IV kann drucklos oder unter Druck, diskontinuierlich oder kontinuierlich durchgeführt werden. Das Sulfondiamid der Formel II kann cyclisiert werden, ohne isoliert zu werden.

Man verwendet unter Reaktionsbedingungen inerte organische Lösungsmittel. Als Lösungsmittel kommen z.B. Halogenkohlenwasserstoffe, insbesondere Chlorkohlenwasserstoffe, z.B.
Tetrachlorethylen,
1,1,2,2- oder 1,1,1,2-Tetrachlorethan,
Dichlorpropan, Methylenchlorid, Dichlorbutan,
Chloroform, Chlornaphthalin, Dichlornaphthalin,
Tetrachlorkohlenstoff,
1,1,1- oder 1,1,2-Trichlorethan,
Trichlorethylen, Pentachlorethan,
o-, m-, p-Difluorbenzol, 1,2-Dichlorethan,
1,1-Dichlorethan, 1,2-cis-Dichlorethylen,
Chlorbenzol, Fluorbenzol, Brombenzol,
Jodbenzol, o-, p- und m-Dichlorbenzol,
o-, p-, m-Dibrombenzol, o-, m-, p-Chlortoluol,
1,2,4-Trichlorbenzol;
Ether, z.B.
Ethylpropylether, Methyl-tert.-butylether,
n-Butylethylether, Di-n-butylether,
Diisobutylether, Diisoamylether,
Diisopropylether, Anisol, Phenetol,
Cyclohexylmethylether, Diethylether,
Ethylenglykoldimethylether, Tetrahydrofuran,

Dioxan, Thionanisol, β,β'-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalls von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3,-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon; und entsprechende Gemische in Betracht. Zweckmässigerweise verwendet man das Lösungsmittel in einer Menge von 100 bis 2000 Gew.%, vorzugsweise von 200 bis 700 Gew.%, bezogen auf Ausgangsstoff III.

Als Säureakzeptoren können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z.B. als basische Verbindungen in Frage: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, α-Picolin, β-Picolin, γ-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Als Cyclisierungsagentien kommen z.B. die vorgenannten anorganischen Säurebindemittel in Frage, ferner Carbonsäurealkalimetallsalze, wie Natriumpropionat, Natriumbutyrat, Natriumisobutyrat, Kaliumformiat, Kaliumacetat, Kaliumpropionat, Kaliumbutyrat, Kaliumisobutyrat, oder Alkalimetallalkoholate, wie Natriummethylat, Natriumethylat, Natriumpropylat, Natriumisopropylat, Natriumbutylat, Natriumisobutylat, Natrium-sec-butylat, Natrium-tert-butylat, Natriumethylenglykolat, Natriumpropylen-(1,2)-glykolat, Natriumpropylen-(1,3)-glykolat, Natriumdiethylenglykolat, Natriumtriethylenglykolat, Natriumdipropylen-(1,2)-glykolat, Kaliummethylat, Kaliumethylat, Kalium-n-propylat, Kaliumisopropylat, Kalium-n-butylat, Kalium-isobutylat, Kalium-sec-butylat, Kalium-tert-butylat, Kaliumethylenglykolat, Kaliumpropylen-(1,2)-glykolat, Kaliumpropylen-(1,3)-glykolat, Kaliumdiethylenglykolat, Kaliumtriethylenglykolat, Kaliumdipropylen-(1,2)-glykolat.

Das Säurebindemittel wird zweckmässig in äquivalenten Mengen oder in einem Überschuss von bis zu 20%, bezogen auf Aminosulfonylhalogenid der Formel IV, eingesetzt.

Die Cyclisierung wird unter Zusatz der 1- bis 2,5-fachen molaren Menge an basischem Cyclisierungsmittel, bezogen auf Sulfondiamid der Formel II, durchgeführt.

Die Ausgangsstoffe der Formeln III und IV werden in ungefähr stöchiometrischem Verhältnis eingesetzt, d.h. in einem Unter- bzw. Überschuss von bis zu 20% Ausgangsstoff der Formel IV, bezogen auf Ausgangsstoff oder Formel III.

Zweckmässigerweise wird das Verfahren so durchgeführt, dass man über zwei Zuführungen das Aminosulfonylhalogenid der Formel IV und die äquivalente Menge an Säureakzeptor bei einer Temperatur zwischen −20 bis +80 °C, vorzugsweise 0 bis 40 °C zu einer ungefähr äquivalenten Menge an N-Carboalkoxyamidin der Formel III in einem inerten organischen Lösungsmittel zulaufen lässt.

Man kann jedoch auch ein Gemisch des Ausgangsstoffs III und des Säureakzeptors in einem inerten organischen Lösungsmittel vorlegen und dann bei −20 bis +80 °C, vorzugsweise bei 0 bis 40 °C, das Aminosulfonylhalogenid der Formel IV zulaufen lassen. Zur Beendigung der Umsetzung rührt man noch 0,5 bis 8 Stunden bei −20 bis +80 °C, vorzugsweise bei 0 bis 40 °C, nach.

Das Reaktionsgemisch wird dann gegebenenfalls eingeengt bzw. im Falle mit Wasser nicht mischbarer Lösungsmittel direkt mit verd. Salzsäure und mit Wasser zur Entfernung der Hydrochloride extrahiert, wobei ein Sulfondiamid der Formel II erhalten wird.

Dieses kann man in wässrigem Medium in Gegenwart der 1- bis 2,5-fachen Menge Base oder auch in organischem Medium in Gegenwart der 1- bis 2,5-fachen Menge Alkoholat zu dem gewünschten 2H-1,2,4,6-Thiatriazin(3)on-1,1,-dioxidsalz cyclisieren. Zur Aufarbeitung säuert man danach an und saugt den ausgefallenen Niederschlag, gegebenenfalls nach weiterem Einengen, ab. Die gewünschten Endstoffe fallen hierbei in reiner Form an, gegebenenfalls können sie durch Umkristallisieren oder Chromatographie gereinigt werden.

Die Verbindungen der Formel Ia liegen, wenn $R^1$ Wasserstoff bedeutet, nach ihren spektroskopischen Daten in der durch die Formel Ia wiedergegebenen Strukturform vor. Je nach Lösungsmittel können dabei jedoch auch gewisse Anteile der tautomeren Form Ia' auftreten, die als im Gleichgewicht befindliche Verbindungen durch die Formel I mitbeansprucht und umfasst werden.

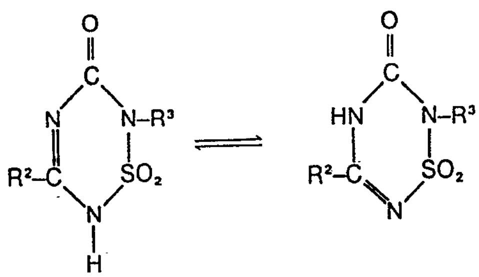

(Ia) (Ia')

Die Verbindung der Formal Ia wirken nicht nur herbizid, sondern sind auch Ausgangsprodukte für die Herstellung neuer selektiver Herbizide. Beispielsweise lassen sich die substituierten 2H-1,2,4,6-Thiatriazin(3)on-1,1-dioxide der Formel Ia durch Umsetzung mit halogenierenden Agentien in 3-Chlor-2H-1,2,4,6-thiatriazin-1,1-dioxide umwandeln, die herbizid wirksam sind.

Beispiel 1

2-Isopropyl-5-methyl-2H-1,2,4,6-thiatriazin-(3)on-1,1-dioxid

101 Gewichtsteile Triethylamin und 157,6 Gewichtsteile Isopropylaminosulfonylchlorid werden über zwei Zuführungen bei 20 bis 25 °C unter Rühren innerhalb 25 Minuten zu 116 Gewichtsteilen N-Carbomethoxy-acetamidin in 550 Volumenteilen Tetrahydrofuran eingeführt. Nach einer Stunde Nachrühren bei 22 °C wird vom ausfallenden Hydrochlorid abgesaugt und mit Tetrahydrofuran nachgewaschen. Anschliessend wird das Filtrat eingeengt, wobei 237 Gewichtsteile (100% d.Th.)
N-Carbomethoxy-N'-isopropylsulfamoyl-acetamidin
als leicht gelbliches Öl vom $n_D^{25} = 1,4890$ erhalten werden.

Dieses Öl wird in 700 Volumenteilen 3n Natronlauge gelöst und 3 Minuten bei 33 °C gerührt. Nun wird die wässrige Lösung einmal mit Methyl-tert.-butylether extrahiert und in eine Mischung von 178 Volumenteilen konz. Salzsäure eingerührt. Nach dem Absaugen, Waschen mit Wasser und Trocknen werden 148 Gewichtsteile (72,2% d.Th., bezogen auf das Ausgangsamidin) farbloses
2-Isopropyl-5-methyl-2H-1,2,4,6-thiatriazin(3)on
vom Fp. 193–196 °C erhalten (Wirkstoff 1).

Beispiel 2

2-Methyl-5-phenyl-2H-1,2,4,6-thiatriazin(3)on-1,1-dioxid

Unter Rühren werden 101 Gewichtsteile Methylaminosulfonylchlorid und 61,7 Gewichtsteile Pyridin über zwei Zuführungen bei 10 bis 20 °C zu 125 Gewichtsteilen N-Carbomethoxy-benzamidin in 700 Volumenteilen Methyl-tert.-butylether gegeben.

Nach 30 Minuten Rühren bei 35 °C wird der entstandene Niederschlag abgesaugt, mit Methyl-tert.-butylether gewaschen und das Filtrat eingeengt. Der Rückstand (190 Gewichtsteile = 100% d.Th.) N-Carbomethoxy-N'-methylsulfamoyl-benzamidin wird in 470 Volumenteilen 3 n Natronlauge gelöst und 10 Minuten bei 30 bis 35 °C gerührt. Anschliessend wird die wässrige Lösung einmal mit Methyl-tert.-butylether extrahiert und in eine Mischung von 121 Volumenteilen konz. Salzsäure in 500 Volumenteilen Eiswasser eingerührt. Nach dem Absaugen, Waschen mit Wasser und Trocknen werden 137 Gewichtsteile (82% d.Th., bezogen auf das Ausgangsamidin) farbloses
2-Methyl-5-phenyl-2H-1,2,4,6-thiatriazin(3)on-1,1-dioxid
vom Fp. 246–249 °C erhalten (Wirkstoff 2).

Beispiel 3

2-Methyl-5-isopropyl-2H-1,2,4,6-thiatriazin-(3)on-1,1-dioxid

136 Gewichtsteile Methylaminosulfonylchlorid und 133 Gewichtsteile N,N-Dimethylcyclohexylamin werden über zwei Zuführungen bei 25 bis 30 °C zu einer Lösung von 114 Gewichtsteilen N-Carbomethoxy-isobutyramidin in 600 Volumenteilen Methylenchlorid gegeben und 30 Minuten bei 20 bis 25 °C gerührt. Die Reaktionsmischung wird einmal mit Wasser extrahiert, getrocknet und eingeengt. Von dem Rückstand (237 Gewichtsteile = 100% d.Th. N-Carbomethoxy-N'-methylsulfamoyl-isobutyramidin) werden 125 Gewichtsteile in einer Lösung von 57,3 Gewichtsteilen Natriumcarbonat in 300 Volumenteilen Wasser gelöst und 3 Minuten bei 40 °C gerührt. Die Lösung wird einmal mit Methyl-tert.-butylether extrahiert und dann parallel mit 92 Volumenteilen konz. Salzsäure über zwei Zuführungen in 100 Teile Eiswasser eingerührt. Nach dem Absaugen, Waschen mit Wasser und Trocknen werden 77 Gewichtsteile (71% d.Th., bezogen auf die eingesetzte Menge Amidin) farbloses

2-Methyl-5-isopropyl-2H-1,2,4,6-thiatriazin-(3)on-1,1-dioxid vom Fp. 194–199 °C erhalten (Wirkstoff 3).

Beispiel 4

2-Methyl-5-isopropyl-2H-1,2,4,6-thiatriazin-(3)on-1,1-dioxid-natriumsalz

3,07 Gewichtsteile 2-Methyl-5-isopropyl-2H-1,2,4,6-thiatriazin-(3)on-1,1,-dioxid werden in einer Lösung von 2,72 Gewichtsteilen 30%igem Natriummethylat in 80 Teilen Methanol gelöst und dann zur Trockne eingeengt. Es werden 3,5 Gewichtsteile (100% d.Th.) des Natriumsalzes des 2-Methyl-5-isopropyl-2H-1,2,4,6-thiatriazin-(3)on-1,1-dioxids vom Fp. 300 °C erhalten (Wirkstoff 4).

Analog werden beispielsweise die folgenden Verbindungen der Formel

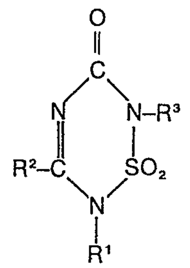

(I),

erhalten:

| Wirkstoff Nr. | $R^1$ | $R^2$ | $R^3$ | Fp[°C] |
|---|---|---|---|---|
| 5 | H | $CH_3$ | H | |
| 6 | H | $CH_3$ | $CH_3$ | 190–193 |
| 7 | $NH_4$ | $CH_3$ | $CH_3$ | |
| 8 | H | $CH_3$ | $C_2H_5$ | 208–210 |
| 9 | Na | $CH_3$ | $C_2H_5$ | |
| 10 | $NH_2(CH_2CH_2OH)_2$ | $CH_3$ | $C_2H_5$ | |
| 11 | H | $CH_3$ | n–$C_3H_7$ | 164–167 |
| 12 | Na | $CH_3$ | n–$C_3H_7$ | 293 (zers.) |
| 13 | Na | $CH_3$ | i–$C_3H_7$ | 298 (zers.) |
| 14 | H | $CH_3$ | n–$C_3H_7$ | |
| 15 | H | $CH_3$ | n–$C_4H_9$ | |
| 16 | Na | $CH_3$ | n–$C_4H_9$ | |
| 17 | H | $CH_3$ | i–$C_4H_9$ | |
| 18 | Na | $CH_3$ | i–$C_4H_9$ | |
| 19 | H | $CH_3$ | sec.–$C_4H_9$ | 154–158 |
| 20 | H | $CH_3$ | tert.–$C_4H_9$ | |
| 21 | Na | $CH_3$ | $C_6H_{11}$ | |
| 22 | H | $CH_3$ | $CH_2CH_2Cl$ | |
| 23 | H | $CH_3$ | –CH(CH₃)–CH(CH₃)–Cl | |
| 24 | H | $CH_3$ | $CH_2CH_2$–O–$CH_3$ | |
| 25 | H | $C_2H_5$ | H | |
| 26 | H | $C_2H_5$ | $CH_3$ | 148–150 |
| 27 | Na | $C_2H_5$ | $CH_3$ | |
| 28 | $NH_2(C_2H_5)_2$ | $C_2H_5$ | $CH_3$ | |
| 29 | H | $C_2H_5$ | $C_2H_5$ | |
| 30 | Na | $C_2H_5$ | $C_2H_5$ | |
| 31 | H | $C_2H_5$ | n–$C_3H_7$ | |
| 32 | H | $C_2H_5$ | i–$C_3h_7$ | |
| 33 | Na | $C_2H_5$ | i–$C_3H_7$ | |
| 34 | H | $C_2H_5$ | N–$C_4H_9$ | |
| 35 | H | $C_2H_5$ | i–$C_4H_9$ | |
| 36 | H | $C_2H_5$ | sek-$C_4H_9$ | |
| 37 | H | $C_2H_5$ | tert.-$C_4H_9$ | |
| 38 | H | $C_2H_5$ | –$CH_2$–$CH_2Cl$ | 205–208 |
| 39 | H | n–$C_3H_7$ | H | |
| 40 | H | n–$C_3H_7$ | $CH_3$ | 160–163 |
| 41 | H | n–$C_3H_7$ | $C_2H_5$ | |
| 42 | H | n–$C_3H_7$ | i–$C_3H_7$ | |
| 43 | H | n–$C_3H_7$ | n–$C_3H_7$ | |
| 44 | H | n–$C_3H_7$ | n–$C_4H_9$ | |

| Wirkstoff Nr. | $R^1$ | $R^2$ | $R^3$ | Fp[°C] |
|---|---|---|---|---|
| 45 | H | $n{-}C_3H_7$ | sec.-$C_4H_9$ | |
| 46 | Na | $n{-}C_3H_7$ | $CH_3$ | |
| 47 | H | $i{-}C_3H_7$ | H | |
| 48 | Na | $i{-}C_3H_7$ | $CH_3$ | |
| 49 | H | $i{-}C_3H_7$ | $C_2H_5$ | |
| 50 | H | $i{-}C_3H_7$ | $n{-}C_3H_7$ | 159–161 |
| 51 | H | $i{-}C_3H_7$ | $i{-}C_3H_7$ | |
| 52 | H | $i{-}C_3H_7$ | sec.-$C_4H_9$ | |
| 53 | H | $n{-}C_4H_9$ | H | |
| 54 | H | $n{-}C_4H_9$ | $CH_3$ | |
| 55 | Na | $n{-}C_4H_9$ | $CH_3$ | |
| 56 | H | $n{-}C_4H_9$ | $C_2H_5$ | |
| 57 | H | $i{-}C_4H_9$ | H | |
| 58 | H | $i{-}C_4H_9$ | $CH_3$ | 168–170 |
| 59 | Na | $i{-}C_4H_9$ | $CH_3$ | >320 |
| 60 | H | $i{-}C_4H_9$ | $C_2H_5$ | |
| 61 | H | $i{-}C_4H_9$ | $n{-}C_3H_7$ | |
| 62 | H | $i{-}C_4H_9$ | $i{-}C_3H_7$ | |
| 63 | H | sec.–$C_4H_9$ | H | |
| 64 | H | sec.–$C_4H_9$ | $CH_3$ | |
| 65 | Na | sec.–$C_4H_9$ | $CH_3$ | |
| 66 | H | Benzyl | H | |
| 67 | H | Benzyl | $CH_3$ | |
| 68 | Na | Benzyl | $CH_3$ | |
| 69 | H | 4-Chlorbenzyl | $CH_3$ | |
| 70 | Na | Phenyl | $CH_3$ | >320 |
| 71 | H | 4-Chlorphenyl | $CH_3$ | |
| 72 | H | 2,4-Dichlorphenyl | $CH_3$ | |
| 73 | H | 4-Isopropylphenyl | $CH_3$ | |
| 74 | H | $CH_2{=}CH$ | $CH_3$ | |
| 75 | H | $CH_2{=}CH{-}CH_2$ | $CH_3$ | |
| 76 | H | $CH_3{-}CH{=}CH{-}CH_2$ | $CH_3$ | |
| 77 | H | $CH_2{=}C(CH_3){-}CH_2$ | $CH_3$ | |
| 78 | H | $H{-}C{\equiv}C{-}CH_2$ | $CH_3$ | |
| 79 | Na | $CH_3$ | $CH_3$ | 310–320 (Zers.) |
| 80 | Na | $CH_3$ | sec.–$C_4H_9$ | 165 (Zers.) |
| 81 | Na | $C_2H_5$ | $ClCH_2{-}CH_2$ | 163 |
| 82 | Na | $i{-}C_3H_7$ | $n{-}C_3H_7$ | 117 (Zers.) |

Die Verbindungen der Formel I bzw. Ia können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen – Fettalkohol – Ether, Alkylsulfonate und Arylsulfonate) und Dispergiermittel, wie Lignin, Sulfitablaugen und Methylcellulose.

Die herbiziden Mittel enthalten im allgemeinen zwischen 0,1 und 95 Gew.%, vorzugsweise zwischen 0,5 und 90 Gew.%, Wirkstoff.

Die Aufwandmengen liegen je nach Zusammensetzung und Wachstumsstadien der Unkrautflora bei 0,1 bis 15 kg und mehr, vorzugsweise zwischen 0,2 und 5 kg Wirkstoff pro Hektar, wobei für

eine totale Pflanzenvernichtung die höheren Aufwandmengen zu verwenden sind.

Die Anwendung kann im Vorauflaufverfahren oder bei Nachauflaufanwendung erfolgen. Vorzugsweise werden die neuen Wirkstoffe vor dem Auflaufen der unerwünschten Pflanzen oder während des Auflaufens derselben sowohl auf Kulturflächen als auch auf unbebautem Land ausgebracht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Beispiele für solche Zubereitungen sind:

I. Man vermischt 90 Gewichtsteile des Wirkstoffs Nr. 1 mit 10 Gewichtsteilen N-Methyl-α-pyrrolidon und erhält eine Lösung, die zur Anwendung in Form kleinster Tropfen geeignet ist.

II. 20 Gewichtsteile des Wirkstoffs Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die 0,02 Gew.% des Wirkstoffs enthält.

III. 20 Gewichtsteile des Wirkstoffs Nr. 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.% des Wirkstoffs enthält.

IV. 20 Gewichtsteile des Wirkstoffs Nr. 4 werden in einer Mischung gelöst, die aus 25 Gewichtsteilen Cyclohexanol, 65 Gewichtsteilen einer Mineralöfraktion vom Siedepunkt 210 bis 280 °C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.% des Wirkstoffs enthält.

V. 20 Gewichtsteile des Wirkstoffs Nr. 26 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselgel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 8 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile des Wirkstoffs Nr. 13 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile des Wirkstoffs Nr. 38 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gew.% Wirkstoff enthält.

IX. 20 Teile des Wirkstoffs Nr. 26 werden mit 2 Teilen Calciumsalz der Dodecylbenzolsulfonsäure, 8 Teilen Fettalkoholpolyglykolether, 2 Teilen Natriumsalz eines Phenolsulfonsäureharnstoffformaldehyd-Kondensates und 68 Teilen eines paraffinischen Mineralöls innig vermischt. Man erhält eine stabile ölige Dispersion.

Die Wirkung der 2H-1,2,4,6-Thiatriazin(3)on-1,1-dioxide der Formel I bzw. Ia auf das Wachstum von unerwünschten und erwünschten Pflanzen wird anhand von Gewächshausversuchen gezeigt.

Als Kulturgefässe dienen Plastikblumentöpfe mit 300 $cm^3$ Inhalt und lehmigem Sand mit etwa 1,5% Humus als Substrat. Bei Galium aparine und Reis wird zusätzlich etwas Torfmull beigemischt, um ein einwandfreies Wachstum zu gewährleisten. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierbei in Wasser als Verteilungsmittel suspendiert und emulgiert und mittels fein verteilender Düsen gespritzt. Die Aufwandmenge beträgt je nach Verbindung 1,0 kg bzw. 2,0 bzw. 4,0 kg Wirkstoff/ha. Nach dem Aufbringen der Mittel werden die Gefässe leicht beregnet, um Keimung und Wachstum in Gang zu bringen. Danach werden die Gefässe mit durchsichtigen Plastikhauben abgedeckt, bis die Pflanzen angewachsen sind. Diese Abdeckung bewirkt ein gleichmässiges Keimen der Testpflanzen, sofern dies nicht durch die Wirkstoffe beeinträchtigt wird.

Zum Zwecke der Nachauflaufbehandlung zog man die Testpflanzen je nach Wuchsform erst bis zu einer Wuchshöhe von 3 bis 15 cm an und behandelte sie danach. Zur Nachauflaufbehandlung werden entweder direkt gesäte und in den gleichen Gefässen aufgewachsene Pflanzen ausgewählt, oder sie werden erst als Keimpflanzen getrennt angezogen und einige Tage vor der Behandlung in die Versuchsgefässe verpflanzt. Die Aufwandmenge für die Nachauflaufbehandlung beträgt 2,0 kg Wirkstoff/ha. Eine Abdeckung unterbleibt bei der Nachauflaufbehandlung.

Die Plastikblumentöpfe werden im Gewächshaus aufgestellt, wobei für wärmeliebende Arten wärmere Bereiche (20 bis 30 °C) und für solche gemässigter Klimate 15 bis 25 °C bevorzugt werden. Die Versuchsperiode erstreckt sich über 3 bis 5 Wochen.

Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Bei diesen Versuchen werden die folgenden Pflanzen getestet:

Amaranthus spp. (zurückgekrümmter Fuchsschwanz), Chenopodium album (Weisser Gänsefuss), Datura stramonium (gemeiner Stechapfel), Galium aparine (Kletterlabkraut), Matricaria spp. (Kamille-Arten), Nicandra physaloides (Giftbeere), Oryza sativa (Reis), Sinapis alba (Weisser Senf), Solanum nigrum (schwarzer Nachtschatten), Zea mays (Mais), Gossypium hirsutum (Baumwolle), Triticum aestivum (Weizen).

Bei der Prüfung der herbiziden Wirkung bei Vorauflaufanwendung erweist sich beispielsweise die Verbindung Nr. 6 bei einer Aufwandmenge von 1,0 kg Wirkstoff/ha als gut wirksam gegen unerwünschte Pflanzen. Bei der Prüfung der herbiziden Wirkung bei Vorauflaufanwendung zeigt die Verbindung Nr. 26 eine gute Wirkung gegen unerwünschte Pflanzen. Ebenso ist beispielsweise Verbindung Nr. 8 mit 4,0 kg/ha wirksam gegen breitblättrige unerwünschte Pflanzen. Wichtige Kulturpflanzenarten werden in selektiver Weise geschont.

Bei der Prüfung der herbiziden Mittel im Nachauflaufverfahren bekämpft beispielsweise die Verbindung Nr. 26 mit 2,0 kg Wirkstoff/ha breitblättrige Unkräuter in selektiver Weise in Kulturpflanzen wie Reis und Baumwolle.

In Anbetracht der Vielseitigkeit der Applikationsmethoden können die Mittel oder diese enthaltende Mischungen noch in einer weiteren grossen Zahl von Kulturen zur Beseitigung unerwünschten Pflanzenwuchses eingesetzt werden.

Im einzelnen seien folgende Nutzpflanzen genannt:

| Botanischer Name | Deutscher Name |
|---|---|
| Allium cepa | Küchenzwiebel |
| Ananas comosus | Ananas |
| Arachis hypogaea | Erdnuss |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weisse Rübe |
| Brassica rapa var. sivestris | Rübsen |
| Camellia sinensis | Teestrauch |
| Carthamus tinctorius | Saflor – Färberdistel |
| Carya illinoinensis | Pekannussbaum |
| Citrus limon | Zitrone |
| Citrus maxima | Pampelmuse |
| Citrus reticulata | Mandarine |
| Citrus sinensis | Apfelsine, Orange |
| Coffea arabica (coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |

| Botanischer Name | Deutscher Name |
|---|---|
| Hordeum vulgare | Gerste |
| Humulus lupulus | Hopfen |
| Ipomoea batatas | Süsskartoffeln |
| Juglans regia | Walnussbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohkohlbenhirse |
| Petroselinum crispum spp. tuberosum | Wurzelpetersilie |
| Picea abies | Rotfichte |
| Abies alba | Weisstanne |
| Pinus spp. | Kiefer |
| Pisum sativum | Gartenerbse |
| Prunus avium | Süsskirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeeren |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preisselbeere |
| Vicia faba | Pferdebohnen |
| Vigna sinensis | Kuhbohne |
| Vitis vinifera | Weinrebe |
| Zea mays | Mais |

Zur Verbreiterung des Wirkungsspektrums und zur Erzielung synergistischer Effekte können in neuen erfindungsgemässen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,2-Benzoxazinderivate, Benzothiadiazinone, 2,6-Dinitroaniline, N-Phenylcarbamate, Thiolcarbamate,

Halogencarbonsäuren, Triazine, Amide, Harnstoffe, Diphenylether, Triazinone, Uracile, Benzofuranderivate, Cyclohexan-1,3-dion-derivate und andere in Betracht.

Ausserdem ist es nützlich, die erfindungsgemässen Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden.

Zur Aktivierung der herbiziden Wirkung können auch Netz- und Haftmittel sowie nicht-phytotoxische Öle und Ölkonzentrate zugesetzt werden.

**Patentansprüche**

1. 2H-1,2,4,6-Thiatriazin(3)on-1,1-dioxide der Formel

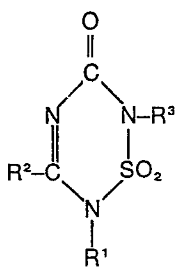

(I),

in der

$R^1$ Wasserstoff, ein Metallatom oder einen gegebenenfalls durch Alkyl oder Hydroxyalkyl mit jeweils 1 bis 10 Kohlenstoffatomen oder Cycloalkyl mit 3 bis 7 Kohlenstoffatomen substituierten Ammoniumrest,

$R^2$ einen unverzweigten $C_1$–$C_{10}$- oder verzweigten Alkylrest, einen Cycloalkylrest mit 3 bis 7 Kohlenstoffatomen, einen halogen-substituierten Alkylrest mit 2 bis 4 Kohlenstoffatomen, Methoxyethyl, Ethoxyethyl, 3-Methoxy-n-propyl, Methoxyisopropyl, 3-Methoxy-n-butyl, 1-Methoxy-butyl-2, Ethoxy-tert-butyl, Methoxy-tert-butyl, 2-Methoxy-butyl, 4-Methoxy-n-butyl, Methylmercapto-ethyl, Ethylmercapto-ethyl, 3-Methylmercapto-n-propyl, 3-Methylmercapto-n-butyl, 1-Methylmercapto-butyl-2, Methylmercapto-tert.-butyl, 2-Methylmercapto-n-butyl oder einen gegebenenfalls durch Halogen oder Alkyl mit 1 bis 4 Kohlenstoffatomen substituierten Phenylrest und

$R^3$ Wasserstoff, einen unverzweigten $C_1$–$C_4$- oder verzweigten $C_3$–$C_4$-Alkylrest, einen Cycloalkylrest mit 3 bis 7 Kohlenstoffatomen, einen halogen-substituierten Alkylrest mit 2 bis 4 Kohlenstoffatomen, Methoxyethyl, Ethoxyethyl, 3-Methoxy-n-propyl, Methoxyisopropyl, 3-Methoxy-n-butyl, 1-Methoxy-butyl-2, Ethoxy-tert-butyl, Methoxy-tert-butyl, 2-Methoxy-butyl oder 4-Methoxy-n-butyl bedeuten,

mit der Massgabe, dass $R^2$ nicht Methyl bedeutet, wenn $R^1$ für Wasserstoff und $R^3$ für Methyl oder Isopropyl stehen, und mit der Massgabe, dass $R^2$ nicht Phenyl bedeutet, wenn $R^1$ für Wasserstoff und $R^3$ für Isopropyl stehen.

2. 2H-1,2,4,6-Thiatriazin(3)on-1,1-dioxide der Formel I gemäss Anspruch 1, dadurch gekennzeichnet, dass $R^1$ für Wasserstoff, $R^2$ für Alkyl mit 1 bis 4 Kohlenstoffatomen und $R^3$ für Alkyl mit 2 bis 4 Kohlenstoffatomen, ausgenommen Isopropyl, stehen.

3. Verfahren zur Herstellung eines 2H-1,2,4,6-Thiatriazin(3)on-1,1-dioxids der Formel I gemäss Anspruch 1, dadurch gekennzeichnet, dass man ein N-Carboalkoxyamidin der Formel

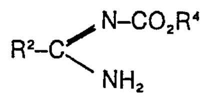

(III),

in der $R^2$ die im Anspruch 1 genannten Bedeutungen hat und $R^4$ einen Alkylrest mit 1 bis 4 Kohlenstoffatomen bedeutet, mit etwa der stöchiometrischen Menge eines Aminosulfonylhalogenids der Formel

$$R^3\text{–}NHSO_2Y \quad (IV),$$

in der $R^3$ die im Anspruch 1 genannten Bedeutungen hat und Y für Fluor oder Chlor steht, gegebenenfalls in Gegenwart eines Säureakzeptors und eines inerten organischen Lösungsmittels bei einer Temperatur im Bereich von −20 bis +80 °C zu einem Sulfondiamid der Formel

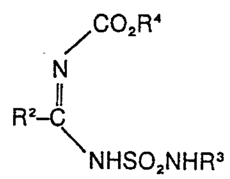

(II),

in der $R^2$ und $R^3$ die im Anspruch 1 genannten Bedeutungen haben und $R^4$ einen Alkylrest mit 1 bis 4 Kohlenstoffatomen bedeutet, umsetzt und dieses in Gegenwart einer basischen Verbindung bei einer Temperatur im Bereich von 0 bis 100 °C cyclisiert.

4. Herbizid, dadurch gekennzeichnet, dass es ein 2H-1,2,4,6-Thiatriazin(3)on-1,1-dioxid der Formel

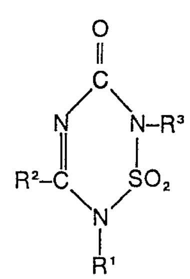

(I),

in der

$R^1$ Wasserstoff, ein Metallatom oder einen gegebenenfalls durch Alkyl oder Hydroxyalkyl mit jeweils 1 bis 10 Kohlenstoffatomen oder Cycloalkyl mit 3 bis 7 Kohlenstoffatomen substituierten Ammoniumrest,

$R^2$ einen unverzweigten $C_1$–$C_{10}$- oder verzweigten $C_3$–$C_{10}$-Alkylrest, einen Cycloalkylrest mit 3 bis 7 Kohlenstoffatomen, einen halogen-substituierten Alkylrest mit 2 bis 4 Kohlenstoffatomen,
Methoxyethyl, Ethoxyethyl, 3-Methoxy-n-propyl,
Methoxyisopropyl, 3-Methoxy-n-butyl,
1-Methoxy-butyl-2, Ethoxy-tert-butyl,
Methoxy-tert-butyl, 2-Methoxy-butyl,
4-Methoxy-n-butyl, Methylmercaptoethyl,
Ethylmercapto-ethyl, 3-Methylmercapto-n-propyl,
3-Methylmercapto-n-butyl,
1-Methylmercapto-butyl-2,
Methylmercapto-tert-butyl,
2-Methylmercapto-n-butyl
oder einen gegebenenfalls durch Halogen oder Alkyl mit 1 bis 4 Kohlenstoffatomen substituierten Phenylrest und

$R^3$ Wasserstoff, einen unverzweigten $C_1$–$C_4$- oder verzweigten $C_3$–$C_4$-Alkylrest, einen Cycloalkylrest mit 3 bis 7 Kohlenstoffatomen, einen halogen-substituierten Alkylrest mit 2 bis 4 Kohlenstoffatomen,
Methoxyethyl, Ethoxyethyl, 3-Methoxy-n-propyl,
Methoxyisopropyl, 3-Methoxy-n-butyl,
1-Methoxy-butyl-2, Ethoxy-tert-butyl,
Methoxy-tert-butyl,
2-Methoxy-butyl oder 4-Methoxy-n-butyl
bedeuten,
mit der Massgabe, dass $R^2$ nicht Methyl bedeutet, wenn $R^1$ für Wasserstoff und $R^3$ für Methyl oder Isopropyl stehen, und mit der Massgabe, dass $R^2$ nicht Phenyl bedeutet, wenn $R^1$ für Wasserstoff und $R^3$ für Isopropyl stehen, enthält.

5. Herbizid, dadurch gekennzeichnet, dass es 2,5-Dimethyl-2H-1,2,4,6-thiatriazin(3)on-1,1-dioxid
als Wirkstoff enthält.

6. Herbizid, dadurch gekennzeichnet, dass es 2-Methyl-5-ethyl-2H-1,2,4,6-thiatriazin(3)on-1,1-dioxid
als Wirkstoff enthält.

7. Herbizid, dadurch gekennzeichnet, dass es 2-Ethyl-5-methyl-2H-1,2,4,6-thiatriazin(2)on-1,1-dioxid
als Wirkstoff enthält.

8. Herbizid, dadurch gekennzeichnet, dass es inerte Zusatzstoffe und ein 2H-1,2,4,6-Thiatriazin(3)on-1,1-dioxid der Formel I gemäss Anspruch 1 enthält, wobei $R^1$ für Wasserstoff, $R^2$ für Alkyl mit 1 bis 4 Kohlenstoffatomen und $R^3$ für Alkyl mit 2 bis 4 Kohlenstoffatomen, ausgenommen Isopropyl, stehen.

**Claims**

1. A 2H-1,2,4,6-thiatrazine-3-one-1,1-dioxide of the formula

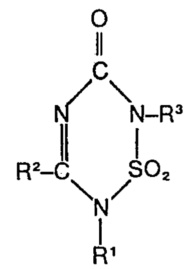

(I),

where

$R^1$ is hydrogen, a metal atom, or ammonium which is unsubstituted or substituted by alkyl or hydroxyalkyl, each of 1 to 10 carbon atons, or by cycloalkyl of 3 to 7 carbon atoms,

$R^2$ is straight-chain $C_1$–$C_{10}$-alkyl or branched $C_3$–$C_{10}$-alky, cycloalkyl of 3 to 7 carbon atoms, halogen-substituted alkyl of 2 to 4 carbon atoms,
methoxyethyl, ethoxyethyl, 3-methoxy-n-propyl,
methoxyisopropyl, 3-methoxy-n-butyl,
1-methoxybut-2-yl, ethoxy-tert-butyl,
methoxy-tert-butyl, 2-methoxybutyl,
4-methoxy-n-butyl, methylmercaptoethyl,
ethylmercaptoethyl, 3-methylmercapto-n-propyl,
3-methylmercapto-n-butyl,
1-methylmercaptobut-2-yl,
methylmercapto-tert-butyl or
2-methylmercapto-n-butyl
or is phenil which is unsubstituted or substituted by halogen or alkyl of 1 to 4 carbon atoms, and

$R^3$ is hydrogen, straight-chain $C_1$–$C_4$-alkyl or branched $C_3$- or $C_4$-alkyl, cycloalkyl of 3 to 7 carbon atoms, halogen-substituted alkyl of 2 to 4 carbon atoms,
methoxyethyl, ethoxyethyl, 3-methoxy-n-propyl,
methoxyisopropyl, 3-methoxy-n-butyl,
1-methoxy-but-2-yl, ethoxy-tert-butyl,
methoxy-tert-butyl,
2-methoxybutyl or 4-methoxy-n-butyl,
with the proviso that $R^2$ is not methyl when $R^1$ is hydrogen and $R^3$ is methyl or isopropyl, and with the proviso that $R^2$ is not phenyl when $R^1$ is hydrogen and $R^3$ is isopropyl.

2. A 2H-1,2,4,6-thiatriazine-3-one-1,1-dioxide of the formula I as claimed in claim 1, wherein $R^1$ is hydrogen, $R^2$ is alkyl of 1 to 4 carbon atoms and $R^3$ is alkyl of 2 to 4 carbon atoms, with the exception of ispropyl.

3. A process for the preparation of a 2H-1,2,4,6-thiatriazine-3-one-1,1-dioxide of the formula I as claimed in claim 1, wherein an N-carboalkoxyamidine of the formula

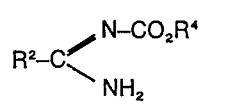

(III),

where $R^2$ has the meanings stated in claim 1 and $R^4$ is alkyl of 1 to 4 carbon atoms, is reacted with about the stoichiometric amount of an aminosulphonyl halide of the formula

$$R^3\text{–}NHSO_2Y \quad (IV)$$

where $R^3$ has the meanings stated in claim 1 and Y is fluorine or chlorine, in the presence or absence of an acid acceptor and of an inert organic solvent, at from −20 to +80 °C, to give sulphonyldiamide of the formula

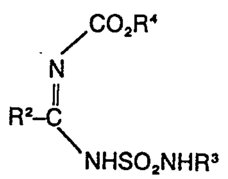

(II),

where $R^2$ and $R^3$ have the meanings stated in claim 1 and $R^4$ is alkyl of 1 to 4 carbon atoms, and this product is cyclized in the presence of a basic compound at from 0 to 100 °C.

4. A herbicide which contains a 2H-1,2,4,6-thiatriazine-3-one-1,1-dioxide of the formula

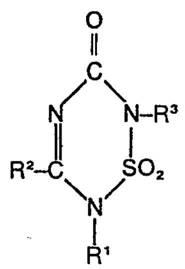

(I),

where

$R^1$ is hydrogen, a metal atom, or ammonium which is unsubstituted or substituted by alkyl or hydroxyalkyl, each of 1 to 10 carbon atoms, or by cycloalkyl of 3 to 7 carbon atoms,

$R^2$ is straight-chain $C_1$–$C_{10}$-alkyl or branched $C_3$–$C_{10}$-alkyl, cycloalkyl of 3 to 7 carbon atoms, halogen-substituted alkyl of 2 to 4 carbon atoms, methoxyethyl, ethoxyethyl, 3-methoxy-n-propyl, methoxyisopropyl, 3-methoxy-n-butyl, 1-methoxybut-2-yl, ethoxy-tert-butyl, methoxy-tert-butyl, 2-methoxybutyl, 4-methoxy-n-butyl, methylmercaptoethyl, ethylmercaptoethyl, 3-methylmercapto-n-propyl, 3-methylmercapto-n-butyl, 1-methylmercaptobut-2-yl, methylmercapto-tert-butyl or 2-methylmercapto-n-butyl or is phenyl which is unsubstituted or substituted by halogen or alkyl of 1 to 4 carbon atoms, and

$R^3$ is hydrogen, straight-chain $C_1$–$C_4$-alkyl or branched $C_3$- or $C_4$-alkyl, cycloalkyl of 3 to 7 carbon atoms, halogen-substituted alkyl of 2 to 4 carbon atoms, methoxyethyl, ethoxyethyl, 3-methoxy-n-propyl, methoxyisopropyl, 3-methoxy-n-butyl, 1-methoxy-but-2-yl, ethoxy-tert-butyl, methoxy-tert-butyl, 2-methoxybutyl or 4-methoxy-n-butyl, with the proviso that $R^2$ is not methyl when $R^1$ is hydrogen and $R^3$ is methyl or isopropyl, and with the proviso that $R^2$ is not phenyl when $R^1$ is hydrogen and $R^3$ is isopropyl.

5. A herbicide which contains 2,5-dimethyl-2H-1,2,4,6,-thiatriazine-3-one-1,1-dioxide as an active ingredient.

6. A herbicide which contains 2-methyl-5-ethyl-2H-1,2,4,6-thiatriazine-3-one-1,1-dioxide as an active ingredient.

7. A herbicide which contains 2-ethyl-5-methyl-2H-1,2,4,6-thiatriazine-2-one-1,1-dioxide as an active ingredient.

8. A herbicide which contains inert additives and a 2H-1,2,4,6-thiatriazine-3-one-1,1-dioxide of the formula I as claimed in claim 1 where $R^1$ is hydrogen, $R^2$ is alkyl of 1 to 4 carbon atoms and $R^3$ is alkyl of 2 to 4 carbon atoms, with the exception of isopropyl.

**Revendications**

1. 1,1-dioxyde de 2H-1,2,4,6-thiatriazin(3)one de formule

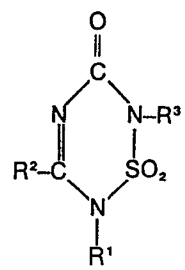

(I),

dans laquelle

$R^1$ représente hydrogène, un atome de métal ou un reste d'ammonium éventuellement substitué par alkyle ou hydroxyalkyle de chacun 1 à 10 atomes de carbone ou cycloalkyle à 3 à 7 atomes de carbone,

$R^2$ représente un reste alkyle ramifié en $C_3$–$C_{10}$ ou non ramifié en $C_1$–$C_{10}$, un reste cycloalkyle à 3 à 7 atomes de carbone, un reste alkyle de 2 à 4 atomes de carbone, substitué par halogène, méthoxyéthyle, éthoxyéthyle, 3-méthoxy-n-propyle, méthoxyisopropyle, 3-méthoxy-n-butyle, 1-méthoxy-butyle-2, éthoxy-tert-butyle, méthoxy-tert-butyle, 2-méthoxy-butyle, 4-méthoxy-n-butyle, méthylmercapto-éthyle, éthylmercapto-éthyle, 3-méthylmercapto-n-propyle, 3-méthylmercapto-n-butyle, 1-méthylmercapto-butyle-2, méthylmercapto-tert-butyle, 2-méthylmercapto-n-butyle, ou un reste phényle éventuellement substitué par halogène ou alkyle à 1 à 4 atomes de carbone, et

$R^3$ représente hydrogène, un reste alkyle ramifié en $C_3$–$C_4$ ou non ramifié en $C_1$–$C_4$, un reste cycloalkyle à 3 à 7 atomes de carbone, un reste alkyle à 2 à 4 atomes de carbone, substitué par halogène, méthoxyéthyle, éthoxyéthyle, 3-méthoxy-n-propyle, méthoxyisopropyle, 3-méthoxy-n-butyle, 1-méthoxy-butyle-2, éthoxy-tert-butyle, méthoxy-tert-butyle, 2-méthoxy-butyle ou 4-méthoxy-n-butyle sous réserve que $R^2$ ne représente pas méthyle quand $R^1$ est mis pour hydrogène et $R^3$ pour

méthyle ou isopropyle et sous réserve que $R^2$ ne représente pas phényle quand $R^1$ est mis pour hydrogène et $R^3$ pour isopropyle.

2. 1,1-dioxide de 2H-1,2,4,6-thiatriazin(3)one de formule I selon la revendication 1, caractérisé par le fait que $R^1$ représente hydrogène, $R^2$ alkyle à 1 é 4 atomes de carbone et $R^3$ alkyle à 2 à 4 atomes de carbone, à l'exclusion de l'isopropyle.

3. Procédé de préparation d'un 1,1-dioxide de 2H-1,2,4,6-thiatriazin(3)one de formule I selon la revendication 1, caractérisé par le fait que l'on fait réagir une N-carboalcoxyamidine de formule

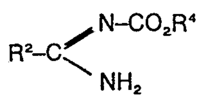

(III),

dans laquelle $R^2$ a la signification donnée dans la revendication 1 et $R^4$ représente un reste alkyle à 1 à 4 atomes de carbone, avec à peu près la quantité stœchiométrique d'un halogénure d'aminosulfonyle de formule

$$R^3\text{–}NHSO_2Y \qquad (IV)$$

dans laquelle $R^3$ a la signification donnée dans la revendication 1 et Y représente fluor ou chlore, éventuellement en présence d'un accepteur d'acide et d'un solvant organique inerte, à une température dans la zone de −20 à +80 °C, pour obtenir un sulfondiamide de formule

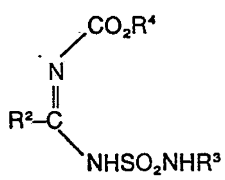

(II),

dans laquelle $R^2$ et $R^3$ ont les significations données dans la revendication 1 et $R^4$ représente un reste alkyle à 1 à 4 atomes de carbone, et on cyclise celui-ci en présence d'un composé basique à une température dans la zone de 0 à 100 °C.

4. Herbicide, caractérisé par le fait qu'il contient du 1,1-dioxide de 2H-1,2,4,6-thiatriazin(3)one de formule

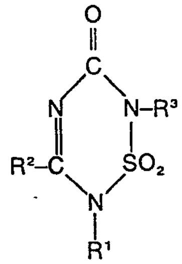

(I),

dans laquelle

$R^1$ représente hydrogène, un atome de métal ou un reste d'ammonium éventuellement substitué par alkyle ou hydroxyalkyle de chacun 1 à 10 atomes de carbone ou cycloalkyle à 3 à 7 atomes de carbone,

$R^2$ représente un reste alkyle ramifié en $C_3$–$C_{10}$ ou non ramifié en $C_1$–$C_{10}$, un reste cycloalkyle à 3 à 7 atomes de carbone, un reste alkyle de 2 à 4 atomes de carbone, substitué par halogène, méthoxyéthyle, éthoxyéthyle, 3-méthoxy-n-propyle, méthoxyisopropyle, 3-méthoxy-n-butyle, 1-méthoxy-butyle-2, éthoxy-tert-butyle, méthoxy-tert-butyle, 2-méthoxy-butyle, 4-méthoxy-n-butyle, méthylmercapto-éthyle, éthylmercapto-éthyle, 3-méthylmercapto-n-propyle, 3-méthylmercapto-n-butyle, 1-méthylmercapto-butyle-2, méthylmercapto-tert-butyle, 2-méthylmercapto-n-butyle, ou un reste phényle éventuellement substitué par halogène ou alkyle à 1 à 4 atomes de carbone, et

$R^3$ représente hydrogène, un reste alkyle ramifié en $C_3$–$C_4$ ou non ramifié en $C_1$–$C_4$, un reste cycloalkyle à 3 à 7 atomes de carbone, un reste alkyle à 2 à 4 atomes de carbone, substitué par halogène, méthoxyéthyle, éthoxyéthyle, 3-méthoxy-n-propyle, méthoxyisopropyle, 3-méthoxy-n-butyle, 1-méthoxy-butyle-2, éthoxy-tert-butyle, méthoxy-tert-butyle, 2-méthoxy-butyle ou 4-méthoxy-n-butyle sous réserve que $R^2$ ne représente pas méthyle quand $R^1$ est mis pur hydrogène et $R^3$ pour méthyle ou isopropyle et sous réserve que $R^2$ ne représente pas phényle quand $R^1$ est mis pour hydrogène et $R^3$ pour isopropyle.

5. Herbicide, caractérisé par le fait qu'il contient, comme principe actif, du 1,1-dioxide de 2,5-diméthyl-2H-1,2,4,6-thiatriazin(3)one.

6. Herbicide, caractérisé par le fait qu'il contient, comme principe actif, du 1,1-dioxide de 2-méthyl-5-éthyl-2H-1,2,4,6-thiatriazin(3)one.

7. Herbicide, caractérisé par le fait qu'il contient, comme principe actif, du 1,1-dioxyde de 2-éthyl-5-méthyl-2H-1,2,4,6-thiatriazin(2)one.

8. Herbicide, caractérisé par le fait qu'il contient des additifs inertes et un 1,1-dioxide de 2H-1,2,4,6-thiatriazin(3)one de formule I selon la revendication 1, $R^1$ représentant hydrogène, $R^2$ alkyle à 1 à 4 atomes de carbone et $R^3$ alkyle à 2 à 4 atomes de carbone, à l'exclusion de l'isopropyle.